(19) Europäisches Patentamt
European Patent Office
Office européen des brevets

(11) Veröffentlichungsnummer: **0 002 014**
**B1**

# (12) EUROPÄISCHE PATENTSCHRIFT

(21) Anmeldenummer: **78101306.5**

(22) Anmeldetag: **04.11.78**

(51) Int. Cl.³: **C 07 C 119/02, A 01 N 37/34**

## (54) Alpha-isocyanocarbonsäure-Salze, Verfahren zu ihrer Herstellung und ihre Verwendung als Pflanzenwachstumsregulatoren.

(30) Priorität: **19.11.77 DE 2751783**

(43) Veröffentlichungstag der Anmeldung:
**30.05.79 Patentblatt 79/11**

(45) Bekanntmachung des Hinweises auf die Patenterteilung:
**28.01.81 Patentblatt 81/4**

(84) Benannte Vertragsstaaten:
**BE CH DE FR GB NL SE**

(56) Entgegenhaltungen:
**DE - A - 2 043 666**
**DE - A - 2 147 707**
**DE - A - 2 218 009**
**DE - A - 2 405 819**

**Chemische Berichte, Jahrg. 109, 1976 (DE) Verlag Chemie, Weinheim I. Hoppe et al. "Cycloaddition von Isocyanketen an Benzophenonanil und Thioimidsäureester zu β - Lactamen", Seiten 482 - 487**

(73) Patentinhaber: **BAYER AG**
**Zentralbereich Patente, Marken und Lizenzen**
**D-5090 Leverkusen 1, Bayerwerk (DE)**

(72) Erfinder: **Schröder, Rolf, Dr.**
**Pahlkestrasse 17**
**D-5600 Wuppertal 1 (DE)**

(72) Erfinder: **Lürssen, Klaus, Dr.**
**August-Kierspel-Strasse 89**
**D-5060 Berg.Gladbach (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 002 014 B1

# $\alpha$-isocyanocarbonsäure-Salze, Verfahren zu ihrer Herstellung und ihre Verwendung als Pflanzenwachstumsregulatoren

Die vorliegende Erfindung betrifft neue $\alpha$-Isocyanocarbonsäure-Salze, ein Verfahren zu ihrer Herstellung sowie ihre Verwendung als Wirkstoffe zur Regulierung des Pflanzenwachstums.

Es ist bereits bekannt geworden, daß Tri-n-butyltrithiophosphorsäureester, 2-Chloräthansulfinsäure und Off-Shoot-T®[1] pflanzenwuchsregulierende Eigenschaften besitzen (vergleiche US-Patentschriften 2 841 486 und 2 965 467, Deutsche Offenlegungsschrift 2 llo 773 und Farm. Chem. Handbook 1975, Meister Publishing Co., Willoughby, Ohio, Pesticide Dictionary D 147).

Die Wirksamkeit dieser Stoffe ist jedoch insbesondere bei niedrigen Aufwandmengen undkonzentrationen nicht immer ganz befriedigend.

Gegenstand dieser Erfindung sind neue $\alpha$-Isocyanocarbonsäure-Salze der Formel

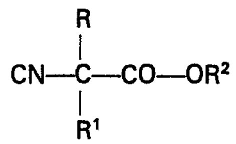 (I)

in welcher

R für Alkyl steht,
$R^1$ für Wasserstoff, Alkyl oder Phenylalkyl steht oder
R und $R^1$ gemeinsam für eine Alkylenkette mit 2 oder 3 Kohlenstoffatomen stehen und
$R^2$ für ein Alkaliion steht,

Gegenstand der Erfindung ist auch ein Verfahren zur Herstellung von $\alpha$-Isocyanocarbonsäure-Salzen der Formel (I) durch Umsetzung von $\alpha$-Isocyanocarbonsäure-alkylestern der Formel

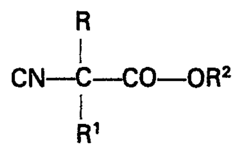 (II)

in welcher

R und $R^1$ die oben angegebene Bedeutung haben und
$R^3$ für Niederalkyl steht
mit einem Alkalihydroxid der Formel

$$HO—R^2 \qquad (III)$$

in welcher

$R^2$ die oben angegebene Bedeutung hat,

gegebenenfalls in Gegenwart eines Lösungs- oder Verdünnungsmittels.

Gegenstand der Erfindung ist ferner die Verwendung der $\alpha$-Isocyanocarbonsäure-Salze der Formel (I) als Wirkstoffe zur Regulierung des Pflanzenwachstums.

Überraschenderweise zeigen die erfindungsgemäßen $\alpha$-Isocyanocarbonsäure-Salze eine erheblich höhere pflanzenwuchsregulierende Wirkung als die aus dem Stand der Technik bekannten Stoffe Tri-n-butyltrithiophosphorsäureester, 2 - Chloräthansulfinsäure und Off-Shoot-T®, welche hochaktive Wirkstoffe gleicher Wirkungsart sind. Die erfindungsgemäßen Stoffe stellen somit eine wertvolle Bereicherung der Technik dar.

Verwendet man beispielsweise $\alpha$-Isocyanpropionsäureäthylester und Kaliumhydroxid als Ausgangsstoffe, so kann der Reaktionsablauf durch das folgende Formelschema wiedergegeben werden:

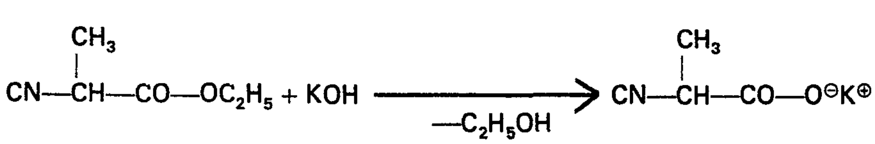


---

1) Off-Shoot-T® ist ein im Handel befindlicher Pflanzenwachstumsregulator auf Basis von Fettalkoholen mit 6, 8, 10 und 12 Kohlenstoffatomen.

Die als Ausgangsstoffe zu verwendenden $\alpha$-Isocyanocarbonsäurealkylester und die Alkalihydroxide sind durch die Formeln (II) und (III) allgemein definiert. Vorzugsweise stehen darin

R für geradkettiges oder verzweigtes Alkyl mit 1 bis 6, insbesondere 1 bis 4 Kohlenstoffatomen,

$R^1$ für Wasserstoff, geradkettiges oder verzweigtes Alkyl mit 1 bis 4, insbesondere 1 oder 2, Kohlenstoffatomen, für Benzyl oder Phenyläthyl oder

R und $R^1$ gemeinsam für eine Alkylenkette mit 2 oder 3 Kohlenstoffatomen,

$R^2$ für ein Natrium- oder Kaliumion und

$R^3$ für Methyl oder Äthyl.

Die erfindungsgemäß verwendbaren $\alpha$-Isocyanocarbonsäurealkylester (II) sind größtenteils bekannt. Die noch nicht vorbeschriebenen Verbindungen der Formel (II) lassen sich nach prinzipiell bekannten Verfahren herstellen. So erhält man zum Beispiel die Methyl- und Äthyl-ester der $\alpha$-Isocyanessig-, $\alpha$-Isocyan-propion- bzw.$\alpha$-Isocyan-valeriansäure nach der von J. Ugi beschriebenen Phosgenmethode (vgl. Angewandte Chemie *77,* 492 ff (1965)) und die am $\alpha$-ständigen Kohlenstoffatom bisalkylierten Verbindungen nach der von Schöllkopf unter anderem im Chem. Ber. *108,* 1580 ff (1975) beschriebenen Methode.

Als Beispiele für die erfindungsgemäß verwendbaren Stoffe der Formel (II) seien im einzelnen genannt:

$\alpha$-Isocyano-propionsäure-, $\alpha$-Isocyano-n-buttersäure-, $\alpha$-Isocyano-n-valeriansäure-, $\alpha$-Isocyano-n-capronsäure-, $\alpha$-Isocyano-iso-valeriansäure-, $\alpha$-Isocyano-iso-buttersäure-, $\alpha$-Isocyano-$\alpha$-methyl-buttersäure-, $\alpha$-Isocyano-$\alpha$- methyl - n - valeriansäure-, $\alpha$-Isocyano-$\alpha$- methyl - capronsäure-, $\alpha$-Isocyano - $\alpha$ - äthyl - n - buttersäure-, $\alpha$-Isocyano - $\alpha$ - äthyl - n - valeriansäure-, $\alpha$-Isocyano - $\alpha$ - phenmethyl - propionsäure- bzw. $\alpha$-Isocyano - $\alpha$ - (2-phenyläthyl) - propionsäuremethyl- bzw. -äthylester und 1-Isocyano - cyclopropylcarbonsäuremethyl bzw. -äthylester.

Die weiterhin als Ausgangsstoffe verwendbaren Alkalihydroxide der Formel (III) sind bekannt. Als Beispiele seien im einzelnen genannt: Natrium und Kaliumhydroxid.

Das Verfahren zur Herstellung der erfindungsgemäßen Verbindungen kann unter Mitverwendung geeigneter Lösungs- oder Verdünnungsmittel durchgeführt werden. Als solche kommen praktisch alle inerten organischen Solventien infrage. Hierzu gehören insbesondere aliphatische oder aromatische, gegebenenfalls chlorierte Kohlenwasserstoffe, wie Benzol, Toluol, Xylol, Methylenchlorid, Chloroform, Tetrachlorkohlenstoff, Chlorbenzol, ferner Äther, wie z.B. Diäthyl- und Dibutyläther und Dioxan, außerdem Aklohole, wie Äthanol. Schließlich kann die erfindungsgemäße Umsetzung auch in Mischungen oben genannter Solventien durchgeführt werden.

Die Reaktionstemperatur kann innerhalb eines größeren Bereichs variiert werden. Im allgemeinen arbeitet man zwischen -10 und 60°C, vorzugsweise bei 0 bis 20°C.

Die erfindungsgemäße Umsetzung wird im allgemeinen bei Normaldruck durchgeführt.

Bei der Durchführung des erfindungsgemäßen Verfahrens zur Darstellung der Verbindungen der Formel (I) setzt man die Alkalihydroxidkomponente (III) vorzugsweise in 10 bis 30%igem Überschuß zu den $\alpha$-Isocyanocarbonsäurealkylestern gegebenenfalls in einem der angegebenen Solventien zu. Nach mehrstündigem Rühren wird abgesaugt, gewaschen und getrocknet.

Die Verbindungen fallen in kristalliner Form an und werden durch ihren Schmelzpunkt charakterisiert.

Als Beispiele für die erfindungsgemäßen $\alpha$-Isocyanocarbonsäure-Salze der Formel (I) seien im einzelnen genannt:

$\alpha$-Isocyano-propionsäure-Natrium
$\alpha$-Isocyano-propionsäure-Kalium
$\alpha$-Isocyano-buttersäure-Natrium
$\alpha$-Isocyano-buttersäure-Kalium
$\alpha$-Isocyano-n-valeriansäure-Natrium
$\alpha$-Isocyano-n-valeriansäure-Kalium
$\alpha$-Isocyano-iso-valeriansäure-Natrium
$\alpha$-Isocyano-iso-valeriansäure-Kalium
$\alpha$-Isocyano-iso-buttersäure-Natrium
$\alpha$-Isocyano-iso-buttersäure-Kalium
$\alpha$-Isocyano-$\alpha$-methyl-buttersäure-Natrium
$\alpha$-Isocyano-$\alpha$-methyl-buttersäure-Kalium
$\alpha$-Isocyano-$\alpha$-äthyl-buttersäure-Natrium
$\alpha$-Isocyano-$\alpha$-äthyl-buttersäure-Kalium
$\alpha$-Isocyano-$\alpha$-phenmethyl-propionsäure-Natrium
$\alpha$-Isocyano-$\alpha$-(2-phenäthyl)-propionsäure-Natrium
$\alpha$-Isocyano-$\alpha$-(2-phenäthyl)-propionsäure-Kalium
$\alpha$-Isocyano-$\alpha$-phenmethyl-propionsäure-Kalium
1-Isocyano-cyclopropylcarbonsaures Natrium
1-Isocyano-cyclopropylcarbonsaures Kalium

Wie bereits mehrfach erwähnt, zeichnen sich die erfindungsgemäßen $\alpha$-Isocyanocarbonsäure-

Salze durch eine pflanzenwuchsregulierende Wirkung aus. Einige Verbindungen weisen auch eine herbizide Wirkung auf.

Aus diesem Grunde können die erfindungsgemäßen Verbindungen mit Erfolg im Pflanzenschutz eingesetzt werden.

Die erfindungsgemäß verwendbaren Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff eine oder auch mehrere verschiedenartige Wirkungen auf Pflanzen ausüben kann. Die Wirkungen der Stoffe hängen im wesentlichen ab von dem Zeitpunkt der Anwendung, bezogen auf das Entwicklungsstadium des Samens oder der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in gewünschter Weise positiv beeinflussen.

Pflanzenwuchsregulierende Stoffe können zum Beispiel zur Hemmung des vegetativen Pflanzenwachstums eingesetzt werden. Eine derartige Wuchshemmung is unter anderem bei Gräsern von wirtschaftlichem Interesse, denn durch eine Dämpfung des Graswachstums kann z.B. die Häufigkeit der Grasschnitte in Ziergärten, Park- und Sportanlagen oder an Straßenrändern reduziert werden. Von Bedeutung ist auch die Hemmung des Wuchses von krautigen und holzigen Pflanzen an Straßenrändern und in der Nähe von Überlandleitungen oder ganz allgemein in Bereichen, in denen ein starker Bewuchs unerwünscht ist.

Wichtig ist auch die Anwendung von Wachstumsregulatoren zur Hemmung des Längenwachstums bei Getreide, denn durch eine Halmverkürzung wird die Gefahr des Umknickens ("Lagerns") der Pflanzen von der Ernte verringert oder vollkommen beseitigt. Außerdem können Waschstumsregulatoren bei Getreide eine Halmverstärkung hervorrufen, die ebenfalls dem Lagern entgegenwirkt.

Eine Hemmung des vegetativen Wachstums erlaubt bei vielen Kulturpflanzen eine dichtere Anpflanzung der Kultur, so daß ein Mehrertrag bezogen auf die Bodenfläche erziet werden kann. Ein weiterer Mechanismus der Ertragssteigerung mit Wuchschemmern beruht darauf, daß die Nährstoffe in stärkerem Maße der Blütenund Fruchtbildung zugute kommen, während das vegetative Wachstum eingeschränkt wird.

Mit Wachstumsregulatoren läßt sich häufig auch eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Föderung des generativen Wachstums führen, so daß z.B. mehr oder größere Früchte zur Ausbildung kommen.

Ertragssteigerungen können in manchen Fällen auch durch einen Eingriff in den pflanzlichen Stoffwechsel erreicht werden, ohne da sich Änderungen des vegetativen Wachstums bemerkbar machen. Wachstumsregulatoren können ferner eine Veränderung der Zusammensetzung der Pflanzen bewirken, um so eine bessere Qualität der Ernteprodukte herbeizuführen. So ist es beispielsweise möglich, den Gehalt an Zucker in Zuckerrüben, Zuckerrohr, Ananas sowie Zitrusfrüchten zu erhöhen oder den Proteingehalt in Soja oder Getreide zu steigern.

Unter dem Einfluß von Wachstumsregulatoren kann es zur Ausbildung parthenokarper Früchte kommen. Ferner kann das Geschlecht der Blüten beeinflußt werden.

Mit Wachstumsregulatoren läßt sich auch die Produktion oder der Abfluß von sekundären Pflanzenstoffen positiv beeinflussen. Als Beispiel sie die Stimulierung des Latexflusses bei Gummibäumen genannt.

Während des Wachstums der Pflanze kann durch Einsatz von Wachstumsregulatoren auch die seitliche Verzweigung durch eine chemische Brechung der Apikaldominanz vermehrt werden. Daran besteht z.B. Interesse bei der Stecklingsvermehrung von Pflanzen. Es ist jedoch auch möglich, das Wachstum der Seitentriebe zu hemmen, z.B. um bei Tabakpflanzen nach der Dekapitierung die Ausbildung von Seitentrieben zu verhindern und damit das Blattwachstum zu fördern.

Unter dem Einfluß von Wachstumsregulatoren kann der Blattbestand von Pflanzen so gesteuert werden, daß ein Entblättern der Pflanzen zu einem gewünschten Zeitpunkt erreicht wird. Eine derartige Entlaubung ist von Interesse, um eine mechanische Beerntung, z.B. bei Wein oder Baumwolle, zu erleichtern oder um die Transpiration zu einem Zeitpunkt herabzusetzen, an dem die Pflanze verpflanzt werden soll.

Durch Einsatz von Wachstumsregulatoren läßt sich der vorzeitige Fruchtfall verhindern. Es ist jedoch auch möglich, den Fruchtfall, — zum Beispiel bei Obst —, im Sinne einer chemischen Ausdünnung bis zu einem bestimmten Ausmaß zu fördern. Wachstumsregulatoren können auch dazu dienen, um bei Kulturpflanzen zum Zeitpunkt der Ernte die zum Ablösen der Früchte erforderliche Kraft zu vermindern, so daß eine mechanische Beerntung der Pflanzen ermöglicht beziehungsweise eine manuelle Beerntung erleichtert wird.

Mit Wachstumsregulatoren läßt sich ferner eine Beschleunigung oder auch eine Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüberhinaus kann mit Hilfe von Wachstumsregulatoren auch eine zeitliche Konzentrierung der Reife erzielt werden. Damit werden die Voraussetzungen dafür geschaffen, daß z.B. bei Tabak, Tomaten oder Kaffee, eine vollständige mechanische oder manuelle Beerntung in nur einem Arbeitsgang vorgenommen werden kann.

Durch Anwendung von Wachstumsregulatoren kann auch die Samen oder Knospenruhe der Pflanzen, also die endogene Jahresrhythmik, beeinflußt werden, so daß die Pflanzen, wie z.B. Ananas oder Zierpflanzen in Gärtnereien, zu einem Zeitpunkt keimen, austreiben oder blühen, an dem sie normalerweise hierzu keine Bereitschaft zeigen.

Mit Wachstumsregulatoren kann auch erreicht werden, daß der Austrieb von Knospen oder die Keimung von Samen verzögert wird, z.B. um in frostgefährdeten Gebieten eine Schädigung durch Spätfröste zu vermeiden.

Wachstumsregulatoren können auch eine Halophilie bei Kulturpflanzen erzeugen. Damit werden die Voraussetzungen dafür geschaffen, daß eine Kultivierung von Pflanzen auf salzhaltigen Böden durchgeführt werden kann.

Mit Wachstumsregulatoren kann auch eine Frost- und Trockenresistenz bei Pflanzen induziert werden.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions - Emulsionskonzentrate, Saatgutpuder, Wirkstoff - imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV -Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Ver-wendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol - Treibgas, wie Halogenkohlenwasserstoffe sowie Butan, Propan, Stickstoff und Kohlendioxid; als feste Trägerstoffe: natürliche Gesteinmehle, wie Kaoline, Tonerden, Talkum, Kreide, Quarz, Attapulgit, Montmorillonit oder Diatomeenerde und synthetische Gesteinmehle, wie hochdisperse Kieselsäure, Aluminiumoxid und Silikate; also feste Trägerstoffe für Granulate: gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehle Kokosnußschalen, Maiskolben und Tabakstengel; aus Emulgierund/oder schaumerzeugende Mittel: nichtionogene und anionische Emulgatoren, wie Polyoxyäthylen-Fettsaüre-Ester, Polyoxyäthylen-Fettalkohol-Äther, z.B. Alkylaryl-polyglykol - äther, Alkylsulfonate, Alkylsulfate, Arylsulfonate sowie Eiweißhydrolysate; als Dispergiermittel: z.B. Lignin — Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo - Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischung mit Düngemitteln und anderen Wachstumsregulatoren.

Die Wirkstoffe können als solche, in Form ihrer Formulierungen oder der daraus bereiteten Anwendungformen, wie gebrauchsfertige Lösungen, emulsierbare Konzentrate, Emulsionen, Schaüme, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Begasen, usw. Es ist ferner möglich, die Wirkstoffe nach dem Ultra - Low - Volume - Verfahren auszubringen, Pflanzen oder Pflanzenteile mit der Wirkstoffzubereitung oder dem Wirkstoff selbst zu bestreichen oder die Wirkstoffzubereitung oder den Wirkstoff selbst in den Boden zu injizieren. Es kann auch das Saatgut der Pflanze behandelt werden.

Die Wirkstoffkonzentrationen können in einem größeren Bereich variiert werden. Im allgemeinen verwendet man pro Hektar Bodenfläche 0,01 bis 50 kg, bevorzugt 0,05 bis 10 kg an Wirkstoff.

Für die Anwendungszeit gilt, daß die Anwendung der Wachstumsregulatoren in einem bevorzugten Zeitraum vorgenommen wird, dessen genaue Abgrenzung sich nach den klimatischen und

vegetarischen Gegebenenheiten richtet.

In den nachfolgenden Beispielen wird die Aktivität der erfindungsgemäßen Stoffe als Wachstumsregulatoren dargestellt, ohne damit die Möglichkeit weiterer Anwendungen als Wachstumsregulatoren auszuschließen.

In den nachfolgenden Beispielen werden die nachstehend angegebenen Verbindungen als Vergleichssubstanzen eingesetzt:

A = $(CH_3—CH_2—CH_2—CH_2—S)_3PO$
(Tri-n-butyl-trithiophosphorsäureester)

B = $Cl—CH_2—CH_2—\overset{\overset{O}{\|}}{S}—OH$
(2-Chloräthansulfinsäure)

C = Off-Shoot-T®
( = Pflanzenwachstumsregulator auf Basis von Fettalkoholen mit 6, 8, 10 und 12 Kohlenstoffatomen).

Beispiel A

*Entlaubung von Baumwollpflanzen bzw. Austrocknen der Blätter bei Baumwolle*

Lösungsmittel: 30 Gewichtsteile Dimethylformamid
Emulgator: 1 Gewichtsteil Polyoxyäthylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Laubblattes angezogen. In diesem Stadium werden die Pflanzen tropfnaß mit den Wirkstoffzubereitungen besprüht. Nach 1 Woche werden der Blattfall und die Austrocknung der Blätter bonitiert. Die Ergebnisse werden mitdenen der unbehandelten Kontrollpflanzen verglichen.

Die erfindungsgemäßen Wirkstoffe 1 und 5 verursachen in diesem Test einen wesentlich stärkeren Blattfall und eine stärkere Austrocknung der Blätter als die aus dem Stand der Technik bekannte Substanz A.

Beispiel B

*Reifebeschleunigung bei Tabak*

Lösungsmittel: 30 Gewichtsteile Dimethylformamid
Emulgator: 1 Gewichtsteil Polyoxyäthylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Tabakpflanzen werden im Gewächshaus bis zur Blüte angezogen. Die Blütenstände werden gekappt. In diesem Stadium werden die Pflanzen tropfnaß mit den Wirkstoffzubereitungen besprüht. Nach 1 Woche wird die Ausfärbung der Blätter im Vergleich zu unbehandelten Kontrollpflanzen bonitiert.

Der erfindungsgemäße Wirkstoff 5 verursacht in diesem Test einen wesentlich schnelleren Reifeverlauf als die aus dem Stand der Technik bekannte Substanz B.

Beispiel C

*Reifebeschleunigung bei Tomaten*

Lösungsmittel: 30 Gewichtsteile Dimethylformamid
Emulgator: 1 Gewichtsteil Polyoxyäthylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Im Freiland werden Tomaten in üblicher Weise angezogen bis etwa die Hälfte der Früchte rot gefärbt ist. In diesem Stadium werden die Pflanzen tropfnaß mit den Wirkstoff-zubereitungen besprüht. Nach 2 Wochen wird die Ausfärbung der Früche bonitiert. Als Vergleich dienen unbehandelte Kontrollpflanzen

Der erfindungsgemäße Wirkstoff 5 verursacht einen wesentlich schnelleren Reifeverlauf als die aus dem Stand der Technik bekannte Substanz B.

Beispiel D

*Hemmung des Seitentriebwachstums bei Tabak*

Lösungsmittel: 30 Gewichtsteile Dimethylformamid

Emulgator: 1 Gewichtsteil Polyoxyäthylen-Sorbitan-Monolaurat

Zur Herstellung einer zweckmäßigen Wirkstoffzubereitung vermischt man 1 Gewichtsteil Wirkstoff mit den angegegebenen Mengen Lösungsmittel und Emulgator und füllt mit Wasser auf die gewünschte Konzentration auf.

Tabakpflanzen werden im Gewächshaus bis zur Entfaltung des 7. Laubblattes angezogen. In diesem Stadium werden die apikalen Vegetationsspitzen der Pflanzen entfernt und die Pflanzen tropfnaß mit den Wirkstoffzubereitungen besprüht. Nach 3 Wochen werden die Sietentriebe der Pflanzen herausgebrochen und gewogen. Das Gewicht der Seitentriebe der behandelten Pflanzen wird mit dem der unbehandelten Kontrollpflanzen verglichen.

Der erfindungsgemäße Wirkstoff 5 verursacht in diesem Test eine wesentlich bessere Hemmung des Seitentriebwachstums als die aus dem Stand der Technik bekannte Substanz C.

Beispiel E

*Wuchshemmung bei Sojabohnen*

Lösungsmittel: 30 Gewichtsteile Dimethylformamid

Emulgator: 1 Gewichtsteil Polyoxyäthylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Sojabohnenpflanzen werden im Gewächshaus bis zur vollen Entfaltung des ersten Folgeblattes angezogen. In diesem Stadium werden die Pflanzen tropfnaß mit den Wirkstoffzubereitungen besprüht. Nach 3 Wochen wird der Zuwachs der behandelten Pflanzen mit dem Zuwachs der unbehandelten Kontrollpflanzen verglichen.

Der erfindungsgemäße Wirkstoff 4 verursacht in diesem Test eine wesentlich stärkere Wuchshemmung als die aus dem Stand der Technik bekannte Substanz B.

Beispiel F

*Wuchshemmung bei Baumwolle*

Lösungsmittel: 30 Gewichtsteile Dimethylformamid

Emulgator: 1 Gewichtsteil Polyoxyäthylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entaltung des 5. Laubblattes angezogen. In diesem Stadium werden die Pflanzen tropfnaß mit den Wirkstoffzubereitungen besprüht. Nach 3 Wochen wird der Zuwachs der behandelten Pflanzen mit dem Zuwachs der unbehandelten Kontrollpflanzen verglichen.

Der erfindungsgemäße Wirkstoff 5 verursacht in diesem Test eine wesentlich stärkere Wuchshemmung als die aus dem Stand der Technik bekannte Substanz B.

Beispiel G

*Wuchshemmung bei Weizen*

Lösungsmittel: 30 Gewichtsteile Dimethylformamid

Emulgator: 1 Gewichtsteil Polyoxyäthylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Weizenpflanzen werden im Gewächshaus bis zum 2 - Blatt - Stadium angezogen. In diesem Stadium werden die Pflanzen tropfnaß mit den Wirkstoffzubereitungen besprüht. Nach 3 Wochen wird der Zuwachs der behandelten Pflanzen mit dem Zuwachs der unbehandelten Kontrollpflanzen verglichen.

Die erfindungsgemäßen Wirkstoffe 1 und 5 verursachen in diesem Test eine wesentlich stärkere Wuchshemmung als die aus dem Stand der Technik bekannte Substanz B.

Beispiel H

*Wuchshemmung bei Gerste*

Lösungsmittel: 30 Gewichtsteile Dimethylformamid

Emulgator: 1 Gewichtsteil Polyoxyäthylen-Sorbitan-Monolaurat

Zur Herstellung einer zweckmäßigen Wirkstoffzubereitung vermischt man 1 Gewichtsteil Wirkstoff mit den angegegebenen Mengen Lösungsmittel und Emulgator und füllt mit Wasser auf die gewünschte Konzentration auf.

Gerstepflanzen werden im Gewächshaus bis zum 2 - Blatt - Stadium angezogen. In diesem Stadium werden die Pflanzen tropfnaß mit den Wirkstoffzubereitungen besprüht. Nach 3 Wochen wird der

Zuwachs der behandelten Pflanzen mit dem Zuwachs der unbehandelten Kontrollpflanzen verglichen.

Der erfindungsgemäße Wirkstoff 5 verursacht in diesem Test eine wesentlich stärkere Wuchshemmung als die aus dem Stand der Technik bekannte Substanz B.

Beispiel I

*Wuchsförderung bei Baumwolle*

Lösungsmittel: 30 Gewichtsteile Dimethylformamid

Emulgator: 1 Gewichtsteil Polyoxyäthylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Laubblattes angezogen. In diesem Stadium werden die Pflanzen tropfnaß mit den Wirkstoffzubereitungen besprüht. Nach 3 Wochen wird der Zuwachs der behandelten Pflanzen mit dem Zuwachs der unbehandelten Kontrollpflanzen verglichen.

Die erfindungsgemäßen Wirkstoffe 1, 3 und 4 verursachen in diesem Test eine starke Förderung des Wachstums.

*Herstellungsbeispiele*

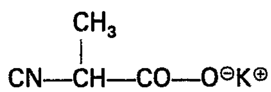

Beispiel 1:

Zu einer Lösung von 50,8 g (0,4 Mol) $\alpha$-Isocyanopropionsäureäthylester in 300 ml Äther tropft man unter Kühlen und Rühren bei ca. 5°C innerhalb von 15 Minuten eine Lösung von 24,6 g (0,44 Mol) Kaliumhydroxid in 500 ml Äthanol. Man rührt anschließend noch 6 Stunden bei Raumtemperatur, saugt ab, spült mit 100 ml Äther nach und entfernt die restlichen Lösungsmittelspuren unter Vakuum im Exsikkator. Es werden 44 g (81% der Theorie) des Kaliumsalzes der $\alpha$- Isocyanopropionsäure in Form eines weißeb Pulvers mit dem Schmelzpunkt 219°C (Zersetzung) isoliert.

Analog Beispiel 1 werden die Verbindungen der Formel

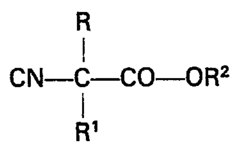 (I)

synthetisiert:

| Beispiel Nr. | R | $R^1$ | $R^2$ | Ausbeute (% der Theorie) | Schmelzpunkt (°C) |
|---|---|---|---|---|---|
| 2 | $C_3H_7$-iso | H | K | 99 | 215 (Zersetzung) |
| 3 | $Ch_3$ | $CH_3$ | K | 69 | 200 |
| 4 | $CH_3$ | 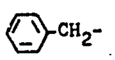 | K | 35 | 235 |
| 5 | $—CH_2—CH_2—$ | | K | 86 | 225 |
| 6 | $—CH_2—CH_2—$ | | Na | 85 | 250 (Zersetzung) |

**Patentansprüche**

1) $\alpha$-Isocyanocarbonsäure-Salze der Formel

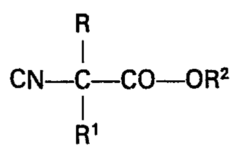 (I)

in welcher

R für Alkyl steht,
$R^1$ für Wasserstoff, Alkyl oder Phenylalkyl steht oder
R und $R^1$ gemeinsam für eine Alkylenkette mit 2 oder 3 Kohlenstoffatomen stehen und
$R^2$ für ein Alkaliion steht.

2) Verfahren zur Herstellung von $\alpha$ - Isocyanocarbonsäure - Salzen, dadurch gekennzeichnet, daß man $\alpha$ - Isocyanocarbonsäure - alkylester der Formel

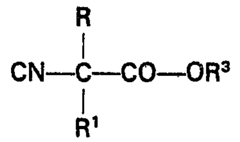

(II)

in welcher

R und $R^1$ die oben angegebene Bedeutung haben und
$R^3$ für Niederalkyl steht,

mit einem Alkalihydroxid der Formel

NO—$R^2$ (III)

in welcher
$R^2$ die oben angegebene Bedeutung hat,

gegebenenfalls in Gegenwart eines Lösungs- oder Verdünnungsmittels umsetzt.

3) Mittel zur Regulierung des Pflanzenwachstums, gekennzeichnet, durch einen Gehalt an mindestens einem $\alpha$ - Isocyanocarbonsäure - Salz gemäß Anspruch 1.

4) Verwendung von $\alpha$ - Isocyanocarbonsäure - Salzen gemäß Anspruch 1 zur Regulierung des Pflanzenwachstums.

**Revendications**

1. Sels d'acides $\alpha$-isocyano-carboxyliques répondant à la formule

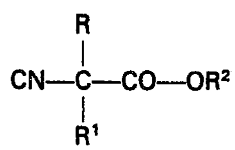

(I)

dans laquelle:
R représente groupe alkyle,
$R^1$ représente l'hydrogène, un groupe alkyle ou phénylalkyle ou bien
R et $R^1$ forment ensemble une chaîne alkylène à 2 ou 3 atomes de carbone, et
$R^2$ représente un ion alcalin.

2. Procédé de préparation de sels d'acides $\alpha$ - isocyanocarboxyliques, caractérisé en ce que l'on fait réagir un ester alkylique d'acide $\alpha$ - isocyano-carboxylique répondant à la formule

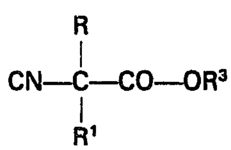

(II)

dans laquelle:

R et $R^1$ ont les significations indiquées ci-dessus et
$R^3$ représente un groupe alkyle inférieur, avec un hydroxyde alcalin répondant à la formule

HO—$R^2$ (III)

dans laquelle:

$R^2$ a les significations indiquées ci-dessus, le cas échéant en présence d'un solvant ou diluant.

3. Produit pour la régulation de la croissance des végétaux, caractérisé en ce qu'il contient au moins un sel d'acide $\alpha$ - isocyano-carboxylique, selon la revendication 1.

4. Utilisation des sels d'acides $\alpha$ - isocyanocarboxyliques selon la revendication 1 pour la régulation de la croissance des végétaux.

## Claims

1) Salts of $\alpha$-isocyanocarboxylic acids of the formula

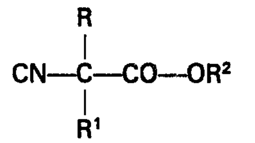

(I)

in which

R represents alkyl,
$R^1$ represents hydrogen, alkyl or phenylalkyl or
R and $R^1$ together represent an alkylene chain with 2 or 3 carbon atoms and
$R^2$ represents an alkali metal ion.

2) Process for the preparation of salts of $\alpha$-isocyano-carboxylic acids, characterised in that $\alpha$-isocyanocarboxylic acid alkyl esters of the formula

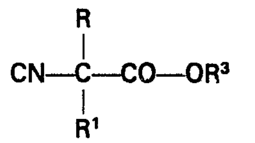

(II)

in which

R and $R^1$ have the meaning indicated above and
$R^3$ represents lower alkyl,
are reacted with an alkali metal hydroxide of the formula

$$HO—R^2 \quad (III)$$

in which

$R^2$ has the meaning indicated above,
optionally in the presence of a solvent or diluent.

3) Agents for regulating plant growth, characterised in that they contain at least one salt, according to Claim 1, of an $\alpha$-isocyanocarboxylic acid.

4) Use of salts, according to Claim 1, of $\alpha$-isocyanocarboxylic acids for regulating plant growth.